# EUROPÄISCHE PATENTSCHRIFT

(11) **EP 1 003 436 B1**
(45) Veröffentlichungstag und Bekanntmachung des Hinweises auf die Patenterteilung: **23.06.2004**
(21) Anmeldenummer: 98935031.9
(22) Anmeldetag: 09.07.1998
(51) Int. Cl.: A61C 3/02, A61C 3/03, A61C 1/14

(54) **MEDIZINISCHES ODER DENTALES INSTRUMENT UND WERKZEUG FÜR EIN SOLCHES INSTRUMENT**
MEDICAL OR DENTAL INSTRUMENT AND A TOOL FOR THE SAME
INSTRUMENT MEDICAL OU DENTAIRE ET OUTIL POUR LEDIT INSTRUMENT

(30) Priorität: 20.08.1997 DE 19736236; 05.06.1998 DE 29810111 U
(43) Veröffentlichungstag der Anmeldung: 31.05.2000
(73) Patentinhaber: Kaltenbach & Voigt GmbH & Co. KG, 88400 Biberach/Riss (DE)
(72) Erfinder: HUGO, Burkhard, D-97265 Hettstadt (DE); MÖSSLE, Walter, D-88441 Mittelbiberach (DE)
(74) Vertreter: Schmidt-Evers, Jürgen, Dipl.-Ing.
(86) Internationale Anmeldenummer: PCT/EP1998/004272
(87) Internationale Veröffentlichungsnummer: WO 1999/008617

(56) Entgegenhaltungen:
- EP-A- 0 399 939
- WO-A-89/09572
- DE-A- 19 510 978
- DE-B- 1 294 592
- DE-C- 299 320
- DE-C- 365 454
- DE-C- 865 781
- DE-U- 29 706 563
- FR-A- 2 632 180
- US-A- 4 295 827

## Beschreibung

Die Erfindung bezieht sich auf ein medizinisches oder dentales Instrument nach dem Oberbegriff des Anspruchs 1.

Zur spanabhebenden Bearbeitung des menschlichen oder tierischen Körpers, insbesondere von Zähnen, oder künstlichen Teilen des Körpers, wie z.B. Prothesen, sind bereits verschiedene Instrumente und zugehörige Werkzeuge bekannt geworden. Grundsätzlich kann man die Arbeitsbewegungen eines vorliegenden Werkzeugs und Instruments in drei Gruppen unterteilen, nämlich Rotationsbewegungen, Longitudinalbewegungen und Schwingbewegungen. Eine Rotationsbewegung findet bei üblichen Bohrern oder Fräsern sowie bei Kreissägen statt. Relativ langhubige Longitudinalbewegungen werden zur Oberflächenbearbeitung angewandt, wie es bei Feilen der Fall ist, wobei die Oberflächenbearbeitung in einer Kavität, z.B. in einem Wurzelkanal oder an einer Außenfläche stattfinden kann, z.B. an der Oberfläche eines Zahns oder Knochens. Es sind auch Kombinationen von vorgenannten Bewegungen möglich und bei Instrumenten und Werkzeugen zur Aufbereitung von Zahnwurzelkanälen auch bereits vorgeschlagen worden. Eine besondere Arbeitsbewegung ist eine kurzhubige Schwingbewegung, bei der es sich sowohl um eine longitudinale Bewegung, um eine in einer Ebene umlaufende, z.B. auf einer kreisförmigen oder ellyptischen Bahn umlaufenden Bewegung, oder um eine räumliche Bewegung handeln kann. Wesentliche Merkmale einer solchen kurzhubigen Schwingbewegung sind eine hohe Frequenz und kleine Amplitude.

Ein Instrument und zugehörige Werkzeuge der zuletzt angegebenen Art sind für dentale Zwecke in der WO 96/14024 beschrieben.

Bei der spanabhebenden Bearbeitung eines vorliegenden Körpers bedarf es gezielter manueller Vorschubbewegungen des Werkzeugs oder Instruments, um z.B. eine bestimmte Form am Körper zu erzeugen oder ein bestimmtes Material am Körper zu entfernen. Ein Beispiel für den ersten Fall ist eine Hinterschneidung einer Kavität, um eine formschlüssige Verankerung einer Füllung zu erreichen. Ein Beispiel für den zweiten Fall ist ein Gewebe mit besonderen Eigenschaften, z.B. ein kariöses Gewebe, das entfernt werden soll, wie es im dentalen Bereich üblich ist. Sofern bei den vorgenannten Maßnahmen eine gute Sicht auf die Behandlungsstelle gewährleistet ist, lassen sich die Vorschubbewegungen verhältnismäßig einfach durchführen und kontrollieren. Insbesondere an solchen Behandlungsstellen, die schlecht oder gar nicht sichtbar sind, z.B. bei einer spanabhebenden Bearbeitung in einer Kavität oder in einer nicht sichtbaren Kavität, gibt es die Möglichkeit, anhand des Widerstandes, den das Gewebe dem Werkzeug entgegensetzt zu erkennen, wie groß die Formveränderung oder die Materialentfernung fortgeschritten ist. Der das Instrument handhabende Arzt entwickelt ein "Gefühl" für die Vorschubtiefe, wobei die Größe des Widerstandes, den ihm das Gewebe bei der Bearbeitung entgegensetzt, ein Indikator für die Eindringtiefe und/oder die Art des Materials ist, was bearbeitet wird. Bei der Kariesentfernung merkt der Arzt bei der Bearbeitung aufgrund des Bearbeitungswiderstandes, ob er sich mit dem Werkzeug im kariösen Gewebe, das verhältnismäßig weich ist, oder im gesunden Gewebe, das eine größere Festigkeit aufweist, befindet.

Bei schneidfreudigen Werkzeugen besteht die Gefahr, daß das Werkzeug bei Ausnutzung des spürbaren erhöhten Widerstandes bereits spanabhebend aktiv ist und das den größeren Widerstand entgegensetzende Gewebe bereits spanabhebend bearbeitet. Bei der Kariesentfernung besteht hierbei die Gefahr, daß neben der erkrankten kariösen Zahnsubstanz auch gesunde Zahnsubstanz entfernt wird. Außerdem gibt es Werkzeuge, deren Andruck am zu bearbeitenden Gewebe dadurch begrenzt ist, daß bei Übersteigung eines bestimmten Andrucks ihre Funktionsfähigkeit beeinträchtigt ist. Ferner besteht insbesondere bei kleinen Werkzeugen die Gefahr einer Beschädigung, insbesondere durch Verbiegen oder Brechen.

Aus US 4 295 827 A ist ein zahnärztliches Instrument der eingangs angegebenen Art zu entnehmen, mit einem Handstück, einer an dessen vorderem Ende angeordneten Haltevorrichtung und einem Wurzelkanal-Werkzeug, das sich quer zum Handstück erstreckt. Die Haltevorrichtung ist durch eine Klemmhülse aus elastischem Material gebildet, die den Werkzeugschaft umgibt und durch eine Klemmmutter radial einwärts gegen den Werkzeugschaft klemmbar ist. Dieses vorbekannte Instrument ist handhabungsunfreundlich, weil es zum Befestigen des Werkzeugschaftes eines manuellen Festziehens der Klemmhülse bedarf, um den Werkzeugschaft am Handstück zu fixieren. Dabei wird die aus elastischem Material bestehende Klemmhülse radial unter Spannung gesetzt, wodurch die Elastizität der Klemmhülse und somit auch die elastisch nachgiebige Lagerung des Werkzeugs beeinträchtigt werden.

In der DE 299 320 C ist ein durch einen Spiralwurzelbohrer gebildetes Werkzeug der eingangs angegebenen Art beschrieben. Das Werkzeug besteht aus einem rosenkopfförmigen spanabhebenden Arbeitskörper, der am freien Ende eines Werkzeugschaftes angeordnet ist, der aus einem das andere Ende bildenden Einspannschaft und einem letzteren mit dem Arbeitskörper verbindenden Verbindungsschaft besteht. Der Verbindungsschaft ist durch eine Wendelfeder gebildet, deren eines Ende mit dem Einspannschaft und deren anderes Ende mit dem Arbeitskörper fest verbunden ist.

Der Erfindung liegt die Aufgabe zugrunde, ein Instrument der eingangs angegebenen Art so auszugestalten, daß ein feinfühliges spanabhebendes Bearbeiten unter Ausnutzung des Arbeitswiderstandes möglich ist.

Diese Aufgabe wird durch die Merkmale des Anspruchs 1 gelöst.

Bei dem erfindungsgemäßen Instrument nach Anspruch 1 ist am Werkzeugschaft wenigstens ein radialer Vorsprung oder wenigstens eine radiale Ausnehmung ausgebildet, wobei der Vorsprung sich elastisch in die Wandung des Verbindungselements hineindrückt oder der Werkstoff des Verbindungselements sich elastisch in die Ausnehmung hineindrückt.

Die Größe der elastischen Rückstellkraft kann für unterschiedliche Werkzeuge und/oder Bearbeitungsmaßnahmen gleich oder unterschiedlich sein. Um zu verhindern, daß das Werkzeug am zu bearbeitenden Gegenstand einen Schaden hervorruft und/oder selbst beschädigt wird, ist es vorteilhaft, die auf den Arbeitskörper wirksame Rückstellkraft kleiner zu bemessen als eine Kraft, die den bzw. die vorgenannten Schäden hervorruft. Bei einem schnittfreudigen Werkzeug, insbesondere bei einem rotierenden Werkzeug wie Bohrer oder Fräser, ist es vorteilhaft, die Rückstellkraft kleiner zu bemessen, als eine Kraft, bei der das Werkzeug beim Vorschub von Hand in das Material des zu bearbeitenden Gegenstandes eindringt und dadurch zu viel Material und/oder eine zu große Kavität erarbeitet wird. Andererseits ist es vorteilhaft, die Rückstellkraft kleiner zu bemessen, als eine vom zu bearbeitenden Gegenstand auf den Arbeitskörper ausgeübte Widerstandskraft, die am Werkzeug einen Schaden hervorrufen kann, z.B. das Werkzeug verbiegen oder brechen kann.

Bei Werkzeugen, die zur spanabhebenden Entfernung von Karies eingesetzt werden, ist es vorteilhaft, die Rückstellkraft gleich oder kleiner als die Widerstandskraft zu bemessen, die bei der Bearbeitung eines Zahns der kariesfreie Schmelz oder insbesondere das kariesfreie Dentin dem Arbeitskörper entgegensetzt. Dabei liegt der Erfindung die Kenntnis zugrunde, daß ein kariesinfiziertes Gewebe weicher ist und sich mit einem geringeren Schnittdruck bearbeiten läßt als ein kariesfreies Gewebe. Wenn deshalb die Rückstellkraft gleich oder kleiner bemessen ist, als die Widerstandskraft, die beim Bearbeiten eines kariesfreien Gewebes erforderlich ist, weicht der bewegbare Arbeitsabschnitt oder das bewegbare Werkzeugteil oder das Werkzeug insgesamt unter dem sich ergebenden Schnittdruck elastisch aus, so daß die vorbeschriebenen Schäden nicht entstehen können.

Die erfindungsgemäße Ausgestaltung eignet sich auch für ein Werkzeug, das eine abrasive Arbeitsfläche aufweist und durch einen Schwingantrieb angetrieben ist, so daß es im Funktionsbetrieb flächige oder räumliche Schwingungen mit einer kleinen Amplitude ausführt. Bei einem solchen Werkzeug besteht die Gefahr, daß dann, wenn der Schnittdruck oder die dadurch hervorgerufene Widerstandskraft des Materials zu groß ist, die Späne von der Arbeitsfläche nicht abtransportiert werden können und dadurch die Leistungsfähigkeit des Werkzeugs abnimmt und beeinträchtigt wird. Ist dagegen die Rückstellkraft so begrenzt, daß der Arbeitsabschnitt oder der Arbeitskörper oder das Werkzeug insgesamt auszuweichen vermag, bevor eine zu große Widerstandskraft gegen die Arbeitsfläche wirkt, dann wird die Funktions und Leistungsfähigkeit des Arbeitskörpers aufrechterhalten, ohne daß der Arzt eine. besondere Aufmerksamkeit auf die Handhabung und die Kraft richten muß, mit der er den Arbeitskörper gegen den zu bearbeitenden Gegenstand drückt. Hierdurch wird auch eine einfachere Handhabung und eine automatische Anpassung des wirksamen Schnittdruckes erreicht.

Bei der erfindungsgemäßen Ausgestaltung kann die Bewegungsrichtung des bewegbaren Arbeitsabschnitts des Arbeitskörpers des Werkzeugs bezüglich der Mittelachse des Schaftfußes des Werkzeugs bzw. der Mittelachse des Instruments unterschiedlich sein, je nachdem, in welcher Position der Arbeitskörper bzw. sein Arbeitsabschnitt angeordnet ist, um eine zu bearbeitende Fläche in bestimmter Position zu erreichen. Da diese Positionen sehr unterschiedlich sein können, insbesondere im Bereich der Zahnreihe bzw. der Zähne im Mundraum eines Patienten, ist es vorteilhaft, eine solche Ausgestaltung der Halterung des Arbeitsabschnitts vorzusehen, bei der der Arbeitsabschnitt jeweils in ein und derselben Bewegungsrichtung oder in unterschiedlichen, z.B. in einem Winkelbereich jeweils unterschiedlichen Bewegungsrichtungen bewegbar ist. Für bestimmte Bearbeitungen kann es noch günstiger sein, eine allseitig bewegbare Halterung vorzusehen, so daß wenigstens der Arbeitsabschnitt des Arbeitskörpers des Werkzeugs oder der Arbeitskörper insgesamt allseitig bewegbar ist. In vielen Einsatzfällen reicht es jedoch bereits aus, die elastische Bewegbarkeit in eine Bewegungsrichtung zu beschränken, z.B. bezüglich der Mittelachse des Instruments oder des Werkzeugschaftfußes axial. Eine allseitige elastische Bewegbarkeit läßt sich in einfacher und vorteilhafter Weise dadurch erreichen, daß der bewegbare Teil des Werkzeugs oder des Werkzeugs insgesamt an einem allseitig elastisch verformbaren Halteteil, z.B. aus Kunststoff oder Gummi gehalten wird. Ein solches elastisch verformbares Teil eignet sich im weiteren auch dazu, eine überdrückbare Klemm- oder Verrastungsvorrichtung zum Halten des Werkzeugteils oder des Werkzeugs insgesamt zu bilden.

Bei allen erfindungsgemäßen Ausgestaltungen wird durch die elastische Bewegbarkeit oder Nachgiebigkeit wenigstens des Arbeitsabschnitts des Arbeitskörpers des Werkzeugs eine Begrenzung des Arbeitsdruckes erreicht, wodurch der zu bearbeitende Gegenstand und/oder der Arbeitsabschnitt bzw. das Werkzeug geschützt werden. Der elastisch bewegbare Arbeitsabschnitt oder der Arbeitskörper insgesamt kann dabei quaderförmig bzw. prismatisch oder zylinderförmig oder kugelförmig oder auch doppelkugelförmig ausgebildet sein. Auch nicht-rotationssymmetrische Arbeitskörper oder Arbeitsansätze wie z.B. "gebogene Kantenformen", mit denen unter sich gehende Zahnbereiche erreichbar sind, können ausgebildet sein.

Die Elastizität der elastischen Rückstellkraft kann je nach Einsatzfall unterschiedlich sein. Bei Versuchen hat sich eine hartelastische Rückstellkraft als vorteilhaft erwiesen.

Es ist im übrigen vorteilhaft, eine Halterung für den erfindungsgemäßen Arbeitsabschnitt oder für den Arbeitskörper oder für das Werkzeug insgesamt vorzusehen, die hinsichtlich der Größe der Rückstellkraft einstellbar ist, so daß unterschiedlich große Rückstellkräfte ausgewählt und eingestellt werden können. Eine einfache Vorrichtung hierfür kann dadurch gebildet sein, daß die Vorspannung der elastischen Rückstellkraft vergrößerbar und verringerbar ist und somit einstellbar ist. Hierzu kann ein Einstellmechanismus am erfindungsgemäßen Werkzeug oder Instrument mit einer Einstellschraube dienen, die gegen ein die elastische Rückstellkraft erzeugendes Federelement spannbar ist und dadurch die Größe der Federkraft verändert.

Die erfindungsgemäße Ausgestaltung eignet sich auch sehr vorteilhaft für kleine Werkzeuge, die aufgrund vorhandener kleiner Querschnitte des Arbeitskörpers oder des Schaftes zum Brechen oder zum Biegen neigen.

Die erfindungsgemäße Ausgestaltung bildet ein Dämpfungsmittel, das die bei der Bearbeitung auftretenden Belastungen auf den zu bearbeitenden Gegenstand oder auf das Werkzeug dämpft. Bezüglich des Handstücks kann das Dämpfungsmittel in der Antriebsverbindung für das Werkzeug angeordnet sein. Bezüglich des Werkzeugs ist das Dämpfungsmittel zwischen dem einen Bearbeitungsabschnitt bildenden Arbeitskörper und dem Werkzeugschaft angeordnet.

Die Erfindung bezieht sich auch auf ein Werkzeug mit einem bezüglich seiner Längsmittelachse vorzugsweise rotationssymmetrischen Arbeitskörper, z.B. mit einem quaderförmigen oder prismatischen oder zylindrischen oder kugelförmigen Arbeitskörper, insbesondere einen sogenannten Rosenbohrer, das sich vorteilhaft zur Bearbeitung von Gewebe des menschlichen oder tierischen Körpers bei einem Schwingantrieb eignet. Es handelt sich um ein an sich bekanntes Werkzeug, das bisher zur spanabhebenden Bearbeitung von Gewebe im rotierenden Funktionsbetrieb eingesetzt wird. Es hat sich gezeigt, daß sich dieses Werkzeug auch im Schwingbetrieb hervorragend eignet und eine handhabungsgünstige Führung des Werkzeugs im Funktionsbetrieb ermöglicht, sowie im weiteren in einfacher Weise zu unzugänglichen Stellen führbar ist, insbesondere dann, wenn der Schaft des Werkzeugs von einer Geraden abweicht und in einer einfachen oder mehrfach abgewinkelten oder entsprechend gebogen verlaufenden Position angeordnet ist. Bei einem Rotationsantrieb ist das Werkzeug auf eine Bauweise eingeschränkt, bei der der Arbeitskörper sich in der Längsmittelachse des Werkzeugs befindet, da andernfalls das Werkzeug nicht rotationsfähig sein würde. Aber auch bei einer Anordnung des Arbeitskörpers in der Längsmittelachse ist ein solches Werkzeug dadurch beeinträchtigt, daß es insbesondere im Bereich der freien Stirnfläche im Zentrum seiner Arbeitsfläche eine kleinere Drehgeschwindigkeit oder Winkelgeschwindigkeit ausführt und deshalb auch die Leistung des Arbeitskörpers in seinem Zentrum beeinträchtigt ist. Bei der erfindungsgemäßen Ausgestaltung ist diese Leistungsbeeinträchtigung dagegen nicht vorhanden, weil die Schwingungsamplituden bei einem rotationssymmetrischen Werkzeug sowohl im Zentrum als auch an der Peripherie des Arbeitskörpers gleich groß sind.

Ein durch einen Schwingantrieb angetriebenes erfindungsgemäßes Werkzeug ist nicht auf eine rotationssymmetrische Bauform eingeschränkt. Der Arbeitskörper kann an einem einfach oder mehrfach abgewinkelten oder entsprechend bogenförmig verlaufenden Schaft angeordnet sein, ohne daß die Abtragsleistung beeinträchtigt wird. Eine solche Bauform ist bei einem durch einen Rotationsantrieb angetriebenen Werkzeug nicht möglich. Ferner ist das erfindungsgemäße Werkzeug dadurch, daß sein Schaft einfach oder mehrfach winkelförmig oder kontrawinkelförmig oder entsprechend gebogen verlaufen kann, auch an unzugänglichen Arbeitsstellen einsetzbar, was insbesondere im Mundraum von Vorteil ist, weil in einem Mundraum eine Ausgangsposition für das Werkzeug durch die Mundöffnung vorgegeben ist.

Ein rotationssymmetrischer Arbeitskörper führt darüber hinaus zu dem Vorteil, daß bei einer Drehung des Arbeitskörpers um seine Mittelachse, z. B. bei einer Drehung in eine andere Arbeitsstellung, die Form der wirksamen Arbeitsfläche gleich bleibt. Besonders vorteilhaft ist eine kugelförmige Form des Arbeitskörpers, weil die Form der wirksamen Arbeitsfläche bei allen möglichen Drehverstellungen bzw. Arbeitsstellungen gleich bleibt, die der behandelnde Arzt oft durchführen kann oder muß. Nichtrotationssymmetrische Arbeitskörper mit entsprechenden Eigenschaften sind z.B. aus der Längsachse abgebogene oder versetzte keulenförmige Instrumente.

Mit einem Werkzeug, das einen bezüglich seines Werkzeugschaftes verdickten Arbeitskörper aufweist, kann eine hinterschnittene Kavität dadurch ausgearbeitet werden, daß das Werkzeug zunächst durch die Öffnung in eine Kavität, die z.B. durch Karies entstanden ist, eingeführt und dann seitlich versetzt wird, so daß der verdickte Arbeitskopf den Öffnungrand der Kavität hintergreift. Beim Vorhandensein solcher Kavitäten ist man bestrebt, die hinterschnittene Form zu belassen und den verjüngten Öffnungsrand nicht zu entfernen, um soviel wie möglich Zahnsubstanz zu erhalten. Es ist. jedoch in vielen Fällen erforderlich, die Fläche der Kavität nicht nur in ihrem hinterschnittenen Bereich sondern auch im Bereich der Kavitätöffnung spanabhebend zu bearbeiten, z.B. um Karies zu entfernen oder eine bestimmte Form zu erhalten. Diese spanabhebende Flächenbearbeitung erfolgt mit dem Arbeitskörper des Werkzeugs. Dies ist nachteilig, weil der verdickte Arbeitskörper eine bestimmte Formgröße vorgibt und deshalb kleinere Formveränderungen im Bereich der Kavitätsöffnung nicht ausgeführt werden können. Außerdem ist eine solche spanabhebende Bearbeitung zeitaufwenig.

Die Abrasivität der Arbeitsfläche am Arbeitskörper kann durch längs und/oder quer verlaufende Schneiden gebildet sein, wie es bei sogenannten Rosenbohrern an sich üblich ist, oder sie kann auch eine durch Vielzahl punktförmiger Schneiden gebildet sein, die auf der jeweiligen Arbeitsfläche dicht verteilt angeordnet sind, wie es bereits beschrieben worden ist. Es ist im Rahmen der Erfindung auch möglich, einen dieser Arbeitsflächenbereiche mit kantenförmigen Schneiden und den anderen Arbeitsflächenbereich mit punktförmigen Schneiden auszustatten. Vorzugsweise sind im Bereich des Arbeitskörpers kantenförmige Schneiden und im Bereich des zusätzlichen Arbeitsflächenabschnitts punktförmige Schneiden vorgesehen, insbesondere eine sogenannte Diamantisierung.

In den Unteransprüchen sind Merkmale enthalten, die zu einfachen, kleinen und kostengünstig herstellbaren sowie gut und sicher funktionierenden Bauweisen sind, die Zugänglichkeit zur Bearbeitungsstelle weiter verbessern und auch zu einer einfachen und die erfindungsgemäße Bewegbarkeit ermöglichenden Halterung des Arbeitsabschnitts oder eines Teils des Werkzeugs oder des Werkzeugs insgesamt ermöglichen.

Nachfolgend werden die Erfindung und weitere durch sie erzielbare Vorteile anhand von vorteilhaften Ausführungsbeispielen und vereinfachten Zeichnung näher erläutert.

Es zeigen
- Fig. 1: ein medizinisches, insbesondere dentales Instrument zur spanabhebenden Bearbeitung von Gewebe des menschlichen oder tierischen Körpers, insbesondere eines Zahns, in der Seitenansicht;
- Fig. 2: das Instrument nach Fig. 1 in abgewandelter Ausgestaltung;
- Fig. 3: den vorderen Endbereich des Instruments in weiter abgewandelter, nicht erfindungsgemäßer Ausgestaltung in der Seitenansicht;
- Fig. 4: den vorderen Endabschnitt eines erfindungsgemäßen Instruments in weiter abgewandelter Ausgestaltung;
- Fig. 5: den vorderen Endabschnitt eines erfindungsgemäßen Instruments in weiter abgewandelter Ausgestaltung;
- Fig. 6: einen Arbeitskörper eines Werkzeugs des Instruments in abgewandelter Ausgestaltung;
- Fig. 7: einen Arbeitskörper des Werkzeugs des Instruments in weiter abgewandelter Ausgestaltung;
- Fig. 8: ein Werkzeug in weiter abgewandelter Ausgestaltung,
- Fig. 9: das Werkzeug nach Fig. 8 in abgewandelter Ausgestaltung;
- Fig. 10: das Werkzeug nach Fig. 8 und im vorderen Endbereich eines erfindungsgemäßen Instruments in der Seitenansicht, teilweise geschnitten;
- Fig. 11: ein Werkzeug nach Fig. 8 im vorderen Endbereich eines Instruments in abgewandelter Ausgestaltung;
- Fig. 12: den vorderen Bereich des Instruments nach Fig. 11 in weiter abgewandelter erfindunggemäßer Ausgestaltung.

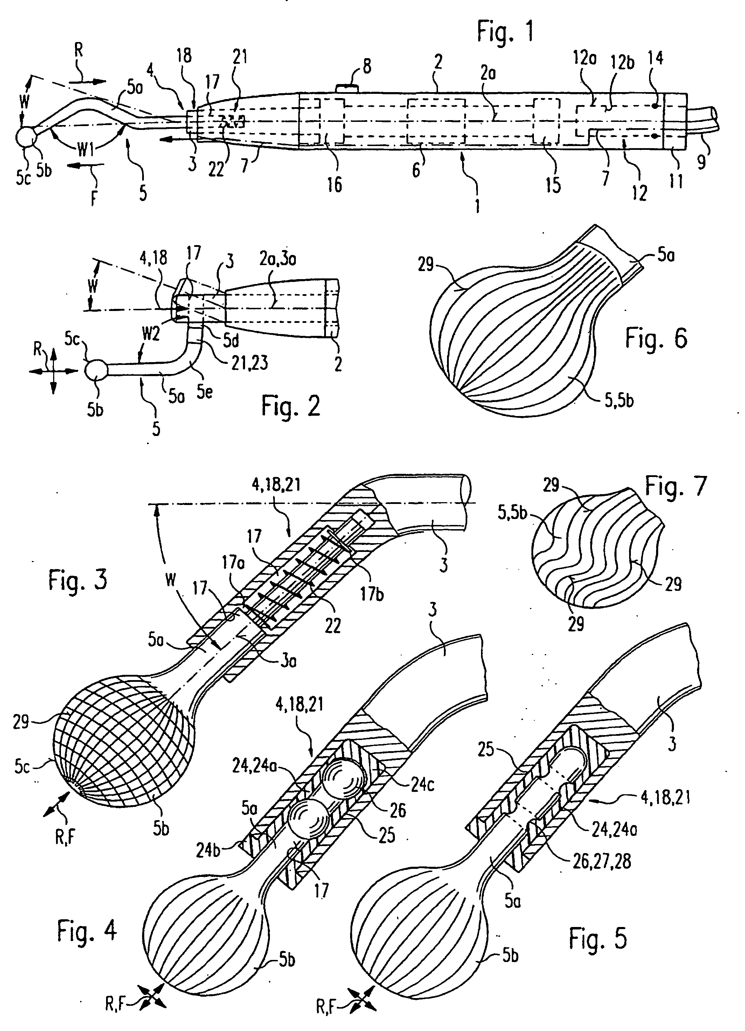

Das in Fig. 1 allgemein mit 1 bezeichnete Instrument eignet sich insbesondere für Knochen-, Zahn- und Kieferbearbeitungen. Die Hauptelemente des Instruments 1 sind ein längliches bzw. stabförmiges Handstück 2 mit einem Handstückschaft 3, der z.B. von dessen vorderem. Ende absteht, in dessen freien Endbereich eine Haltevorrichtung 4 angeordnet ist, in der ein Werkzeug 5 mit einem Werkzeugschaft 5a und einem davon abstehenden Arbeitskörper 5b lösbar gehalten ist, ein Vibrationsantrieb 6 für den Handstückschaft 3, der im Handstück 2 angeordnet ist, und vorzugsweise auch eine elektronische Steuereinrichtung zur Vergrößerung oder Verringerung der Antriebsleistung, wobei die Steuereinrichtung im Instrument 1 bzw. Handstück 2 oder auch entfernt davon, z.B. an einem nicht dargestellten Steuergerät oder einem Fußschalter angeordnet sein kann. Vorzugsweise ist zur Einstellung der gewünschten Antriebsleistung ein allgemein mit 8 bezeichnetes Einstellglied vorgesehen, das bei der vorliegenden Ausgestaltung an der Mantelfläche des Handstücks 2 angeordnet ist und dort verschiebbar gelagert ist, jedoch auch vom Handstück 2 oder Instrument 1 entfernt angeordnet sein kann.

Das Instrument 1 ist durch eine andeutungsweise dargestellte flexible Versorgungsleitung 9 mit einem flexiblen Versorgungsschlauch mit dem Steuergerät verbunden, wobei in oder an der Versorgungsleitung 9 eine oder mehrere Medienleitungen 7 zur Versorgung des Instruments 1 mit Energie und Behandlungs- und/oder Bearbeitungsmedien verlaufen.

Bei der vorliegenden Ausgestaltung besteht das Instrument 1 aus dem ein vorderes Instrumententeil bildenden Handstück 2 und einem ein hinteres Instrumententeil bildendes Anschlußstück 11, das an seinem hinteren Ende mit der flexiblen Versorgungsleitung 9 verbunden ist und durch eine Schnellschlußkupplung 12, insbesondere eine Steck- oder eine Schraubkupplung, mit dem Handstück 2 lösbar verbunden ist. Bei der Schnellschlußkupplung 12 handelt es sich vorzugsweise um eine solche, die im gekuppelten Zustand ein Drehen des Handstücks 2 um seine Längsmittelachse 2a und dabei den Durchgang des oder der vorhandenen Medien gewährleistet. Bei der vorliegenden Ausgestaltung ist eine Steckkupplung mit einem zylindrischen oder stufenzylindrischen Kupplungszapfen 12a und eine ihn drehbar aufnehmende Kupplungsausnehmung 12b vorgesehen, wobei beim vorliegenden Ausführungsbeispiel der Kupplungszapfen 12a vom Anschlußstück 11 nach vorne absteht und die Kupplungsausnehmung 12b nach hinten aus dem Handstück 2 ausmündet. Durch eine ansich bekannte lösbare, insbesondere manuell überdrückbare elastische Sicherungsvorrichtung 14 ist im gekuppelten Zustand ein unbeabsichtigtes Lösen der Steckkupplung verhindert. Für einen Trennvorgang läßt sich die mit einem elastisch vorgespannten Sicherungselement wirksame Sicherungsvorrichtung 14 handhabungsfreundlich überdrücken und lösen.

Der Handstückschaft 3 ist im Handstück 2 allseitig elastisch schwingbar gelagert. Hierzu können elastisch nachgiebige bzw. kompremierbare Lagerteile, z.B. Lagerringe, dienen, von denen zwei in einem axialen Abstand voneinander angeordnet und andeutungsweise dargestellt sind. Das eine Lagerteil 15 kann radial elastisch nachgiebig sein, während das vorzugsweise vordere Lagerteil 16 radial und axial nachgiebig ist. Aufgrund der elastisch nachgiebigen Ausbildung wird der Handstückschaft 3 durch die Elastizität der Lagerteile 15, 16 im Ruhezustand in eine Vibrations-Mittelstellung zurückgestellt. Der Schwingungserzeuger bzw. Vibrationsantrieb 6 erzeugt hochfrequente kurzhubige Schwingungen im Sinne einer Vibration mit einer vorzugsweise im Schall- oder Ultraschallbereich liegenden Frequenz, wobei die Schwingungen bzw. Amplituden z.B. quer und/oder längs des Handstückschaftes 3 linear gerichtet sein können oder ellipsen- oder kreisförmig umlaufend sein können und zwar jeweils in einer Ebene oder ihre Richtung wechselnd räumlich verlaufen können. Räumlich umlaufende Schwingungen haben sich als vorteilhaft erwiesen. Aufgrund der radialen und axialen elastisch nachgiebigen Lagerung des Handstückschaftes 3 stellen sich im Funktionsbetrieb räumliche Schwingungen ein, so daß das Werkzeug 5 in allen Richtungen abrasiv wirksam ist. Die jeweilige Wirkrichtung des Werkzeugs 5, in der sich letzteres in das zu bearbeitende Material einsenkt, ist quer, vorzugsweise rechtwinklig, zur abrasiven Arbeitsfläche 5c des Werkzeugs 5 gerichtet.

Beim vorliegenden Ausführungsbeispiel weist der Vibrationsantrieb eine Frequenz von etwa 4 kHz bis 8 kHz, vorzugsweise etwa 6 kHz, auf, wobei sich im Bereich des Werkzeugs 5 eine Amplitude der vorzugsweise räumlichen Schwingungen von etwa 0,05 mm bis 0,2 mm oder bis 0,5 mm, insbesondere etwa 0,1 mm, ergibt. Dabei kann die Steuereinrichtung so ausgebildet sein, die sie eine Einstellung der Schwingungsleistung im vorgenannten Bereich oder auch eine Einstellung über diesen Bereich hinaus ermöglicht, so daß gegebenenfalls auch beträchtlich größere Amplituden einstellbar sein können.

Das erfindungsgemäße Instrument 1 eignet sich deshalb besonders gut für unterschiedliche Werkzeuge 5, die als Werkzeug-Sortiment zugeordnet sein können und sich aufgrund unterschiedlicher Form und/oder Größe und/oder Zweckbestimmung voneinander unterscheiden.

Die Haltevorrichtung 4 weist ein Steckloch 17 im Handstückschaft 3 für den vorzugsweise zylindrischen Werkzeugschaft 5a auf und eine Fixiervorrichtung 18 für den Werkzeugschaft 5a, die ihn im Steckloch 17 sichert und starr oder so elastisch nachgiebig sein kann, daß bei Überschreitung einer am Werkzeug 5 in Umfangsrichtung und/oder axial wirksamen Belastungskraft die Fixiervorrichtung 18 selbsttätig nachgibt, wodurch eine Beschädigung oder ein Abbrechen des Werkzeugs 5 vermieden wird. Aus Vereinfachungsgründen ist bei den vorliegenden Ausführungsbeispielen die Fixiervorrichtung 18 als Pfeil dargestellt. Es kann sich um eine in den Handstückschaft 3 radial eingeschraubte Klemmschraube oder eine elastisch vorgespannte Rundung handeln, die jeweils in eine Kalotte (nicht dargestellt) im Werkzeugschaft 5a einfassen.

Die Haltevorrichtung 4 bildet eine lösbare Befestigungsvorrichtung für das Werkzeug 5. Da die Befestigung jedoch starr oder elastisch nachgiebig oder durch manuelle Einschub- oder Auszugkräfte überdrückbar sein kann, wird die Befestigungsvorrichtung mit Haltevorrichtung 4 bezeichnet.

Der im Querschnitt vorzugsweise runde und insbesondere axial vom Handstück 2 abstehende Handstückschaft 3 kann sich gemäß Fig. 1 gerade erstrecken oder in seinem vorderen Endbereich abgewinkelt sein, z.B. um einen Winkel W von etwa 5 bis 60°, insbesondere etwa 15°. Das Steckloch 17 kann sich axial (Fig. 1) oder quer, insbesondere rechtwinklig (Fig.2), im Handstückschaft 3 befinden, wobei der Arbeiskörper 5b in der Handstückachse 2a, z.B. (Fig. 1) oder davon quer, versetzt, z.B. (Fig. 2) angeordnet sein kann.

Bereits die vorbeschriebenen Ausgestaltungen des Handstückschafts 2 und/oder des Werkzeugs 5 ermöglichen eine Vielzahl von Werkzeugstellungen bezüglich des Instruments 1 bzw. des Handstückschafts 3, so daß auch in schwierigen Bearbeitungsstellungen am zu bearbeitenden Körper, z.B. im Mundraum, eine gute Zugänglichkeit gewährleistet ist.

Das Werkzeug 5 kann um die Längsmittelachse des Werkzeugschaftes 5a in Stufen oder stufenlos drehbar und einstellbar in der Haltevorrichtung 4 positionierbar sein. Hierdurch wird das Instrument 1 oder das Werkzeug 5 hinsichtlich der Eignung für unzugängliche Bearbeitungsstellen weiter verbessert. Außerdem eignet sich ein und dasselbe Instrument 1 für Werkzeuge 5, die sich eine unterschiedlichen Größe und/oder Form und/oder Abrasivität der Arbeitsfläche 5c, z.B. grob und fein oder grob oder mittel und fein, voneinander unterscheiden und ein Werkzeug-Set, bilden, mit dem das Instrument ausrüstbar ist.

Bei der Ausgestaltung nach Fig. 1 ist der Werkzeugschaft 5a bogenförmig oder knickförmig seitlich ausgeformt, wobei die den Winkel der Ausbiegung oder den Knick bildenden Schenkel gleich oder unterschiedlich sein können. Der Winkel W1 zwischen den Schenkeln kann etwa 60 bis 120°, insbesondere etwa 90°, betragen.

Bei der Ausgestaltung nach Fig. 2 kann der von den Schaftschenkeln eingeschlossene Winkel W2 ein rechter Winkel sein oder größer oder kleiner, bemessen sein, z.B. im Winkelbereich von etwa 60 bis 120°.

Bezöglich des Intruments 1 werden mehrere Ausführungsbeispiele beschrieben, deren Merkmale nicht auf das jeweilige Ausführungsbeispiel beschränkt sind sondern wechselseitig mit den anderen Ausführungsbeispielen kombiniert werden können. Die elastische Verstellbarkeit oder elastische Nachgiebigkeit wenigstens eines Arbeitsabschnitts des Werkzeugs 5 ist durch eine elastische Verbindungsvorrichtung gewährleistet, die allgemein mit 21 bezeichnet ist, wobei sie in die Haltevorrichtung 4 integriert sein kann, also zwischen dem Werkzeug 5 und dem Handstück 2 angeordnet sein kann (Fig. 1) oder zwischen zwei Werkzeugschaftteilen 5d, 5e angeordnet sein kann (Fig. 2). Dabei kann die Richtung R der elastischen Bewegbarkeit oder die elastische Nachgiebigkeit jeweils in einer oder in mehreren Richtungen, z.B. in der Längsrichtung des Werkzeugschaftes 5a oder des Handstückschaftes 3, z.B. im Sinne einer Teleskopierbarkeit, oder quer dazu oder allseitig, d.h. räumlich, verlaufen.

Bei der Ausgestaltung nach Fig. 1 ist die Verbindungsvorrichtung 21 in die Haltevorrichtung 4 integriert, wobei eine elastische Bewegbarkeit in einer Richtung, nämlich in der Längsrichtung des Handstücks 2 vorgesehen ist. Die elastische Verbindungsvorrichtung 21 kann durch eine Feder 22, z.B. eine Wendelfeder, gebildet sein, die im Steckloch 17 zwischen dem Werkzeugschaft 5a und dem Handstückschaft 3 angeordnet ist, wodurch eine elastische Bewegbarkeit für das Werkzeug 5 in axialer Richtung auf das Handstück 2 hin gegeben ist. Dies kann dadurch gewährleistet sein, daß der Handstückschaft 5a im Steckloch 17 längs verschiebbar und vorzugsweise durch eine Drehsicherung drehgesichert aufgenommen ist. Zur Sicherung des Werkzeugschaftes 5a gegen ein unbeabsichtigtes Herausziehen aus dem Steckloch 17 kann der hier begrenzt beweglichen Fixiervorrichtung 18 eine Sicherungsvorrichtung, z.B. ein Anschlag, vorgesehen sein.

Bei der Ausgestaltung nach Fig. 2, bei der gleiche oder vergleichbare Teile mit den gleichen Bezugszeichen versehen sind, ist die elastische Verbindungsvorrichtung 21 durch ein elastisch verformbares Verbindungselement 23 gebildet, das die Werkzeugschaftteile 5d, 5e miteinander verbindet, so daß der Werkzeugschaftteil 5e relativ zum Werkzeugschaftteil 5d elastisch bewegbar ist. Das elastische Verbindungselement 23 kann z.B durch einen dritten Werkzeugschaftabschnitt aus elastisch verformbaren Material, z.B. Gummi oder Kunststoff, gebildet sein, das mit den Werkzeugschaftteilen 5ad, 5ae verbunden, z.B. verklebt ist. Bei einer solchen Ausgestaltung ist der Werkzeugschaftteil 5e relativ zum Werkzeugschaftteil 5d allseitig schwenkbar und gegebenenfalls in der Längsrichtung des Werkzeugschaftes 5 gegen eine elastische Rückstellkraft F bewegbar und zwar in beide Längsrichtungen des Werkzeugschaftteiles 5d.

Außerdem ist bei der Ausgestaltung nach Fig. 2 das Steckloch 17 quer, hier z.B. rechtwinklig, zur Längsachse 3a des Handstückschaftes 3 gerichtet, wobei sich der Werkzeugschaft 5a gerade oder abgewinkelt oder abgebogen erstrecken kann, s. Winkel W2.

Beim Ausführungsbeispiel nach Fig. 3, bei dem gleiche oder vergleichbare Teile ebenfalls mit gleichen Bezugszeichen versehen sind, ist die Verbindungsvorrichtung 21 zwar ebenfalls zwischen dem Werkzeug 5 und dem Handstückschaft 3 angeordnet, wobei sie eine relative Bewegbarkeit des Werkzeugs 5 in der Längsrichtung des Handstückschaftes 3 ermöglicht, jedoch ist der Handstückschaft 3 um den Winkel W abgewinkelt, so daß die Bewegungsrichtung einen spitzen Winkel mit der Längsrichtung des Handstücks 2 einschließt. Bei dieser Ausgestaltung ist ein Steckloch 17 im Handstückschaft 3 angeordnet, wobei der Werkzeugschaft 5a im hier axialen Steckloch 17 verschiebbar gelagert ist und zwischen dem Werkzeugschaft 5a und dem Handstückschaft 3 eine Feder 22 angeordnet ist. Die Feder 22 kann so am Handstückschaft 3 abgestützt sein und am Werkzeugschaft 5a angreifen, daß die elastische Rückstellkraft F in die Einschubrichtung oder in die Ausschubrichtung gerichtet ist. Bei der Ausgestaltung nach Fig. 3 ist beispielhaft eine Anordnung vorgesehen, bei der die Feder 22 mit ihrem vorderen Ende an einem Anschlag 17a eines stufenförmigen Stecklochs 17 abgestützt ist und mit ihrem hinteren Ende gegen einen zweiten Anschlag 17b am Werkzeugschaft 5a, z.B. an einem sogenannter Seegerring, angreift und das Werkzeug 5 in die Einschubrichtung vorspannt. Bei dieser Ausgestaltung ist das Werkzeug 5 in die Ausschubrichtung elastsich nachgiebig. Dies ist insbesondere bei einem Werkzeug 5 mit einem kopfförmig verdickten Arbeitskörper 5b von Bedeutung. Bei dieser Ausgestaltung ist die durch die Federkraft begrenzte, am zu bearbeitenden Gegenstand wirksame Andruckkraft nach hinten bzw. in der Längsrichtung des Werkzeugschafts 3 in Richtung auf das Handstück 2 gerichtet. Es ist im Rahmen der Erfindung auch möglich, den Anschlag gemäß Fig. 1 oder den Anschlag 17b am Werkzeugschaft 5a zwischen zwei beiderseits am Handstückschaft 3 abgestützten Federn 17 anzuordnen, so daß das Werkzeug 5 mittenzentriert in beide Längsrichtungen des Werkzeugschafts 3 elastisch nachgiebig ist und somit auch die elastische Rückstellkraft F in beide Längsrichtungen des Werkzeugschafts 3 wirksam ist. Dies ist durch einen die Rückstellkraft F verdeutlichenden Doppelpfeil veranschaulicht.

Bei den Ausgestaltungen nach den Fig. 4 und 5 weist die elastische Verbindungsvorrichtung 21 ein elastisch verformbares Verbindungselement 24 auf, das den Handstückschaft 3 mit dem Werkzeugschaft 5a verbindet, so das letzterer gegen eine Rückstellkraft elastisch bewegbar bzw. nachgiebig am Werkzeugschaft 3 gelagert ist. Das Verbindungselement 24 weist im wesentlichen die Form einer vorzugsweise runden Hülse 24a auf, die in ein Aufnahmeloch 25 des Handstückschafts 3 eingesetzt ist und bei der Ausgestaltung gemäß Fig. 4 und 5 darin fixiert ist, z.B. durch Pressitz oder Kleben. Bei dieser Ausgestaltung bildet die Hülse 24a das Steckloch 17, in das der Werkzeugschaft 5a einsteckbar und elastisch bewegbar bzw. nachgiebig gehalten ist. Die axiale Halterung kann durch wenigstens einen radialen Vorsprung 26 oder wenigstens eine radiale Ausnehmung 27 am Werkzeugschaft 5a gebildet sein, wobei der radiale Vorsprung 26 sich elastisch in die Wandung der Hülse 24a hineindrückt oder der Werkstoff der Hülse 24a elastisch in die radiale Ausnehmung 27 hineindrückt. Um das Ein- und Ausschieben des Werkzeugschafts 5a zu erleichtern, ist der wenigstens eine Vorsprung 26 und die wenigstens eine Ausnehmung 27 vorzugsweise gerundet. Es ist außerdem vorteilhaft, wenn der Vorsprung 26 oder die Ausnehmung 27 sich ringförmig auf dem gesamten Umfang des Werkzeugschaftes 5a erstrecken. Es können auch wenigstens zwei Vorsprünge 26 oder Ausnehmungen 27 axial hintereinander angeordnet sein. Bei der Ausgestaltung nach Fig. 4 ist der wenigstens eine Vorsprung 26 durch eine kugelförmige Verdickung gebildet. Bei der Ausgestaltung nach Fig. 5 ist die wenigstens eine Ausnehmung 27 durch eine Ringnut gebildet.

Die Hülse 24a kann an ihrem vorderen Rand einen Flansch 24b aufweisen, mit dem sie am vorderen Ende des Handstückschafts 3 anliegt. An ihrem hinteren Ende kann die Anschlußhülse 24a einen Hülsenboden 24c aufweisen, an dem der Werkzeugschaft 5a anliegen kann. Der Umfangsrand am freien Ende des Werkzeugschafts 5a ist vorzugsweise gerundet, um das Einschieben in die Hülse 24a zu erleichtern. Bei der Ausgestaltung nach Fig. 4 kann die Querschnittsgröße des Stecklochs 17 der Querschnittsgröße des Werkzeugschaftes 5a entsprechen. Bei der Ausgestaltung nach Fig. 5 kann die Querschnittsgröße des Stecklochs 17 kleiner bemessen sein als die Querschnittsgröße des Werkzeugschafts 5a, so daß das Material der Hülse 24a sich in die wenigstens eine Ausnehmung 27 hineindrückt, oder die Hülse 24a kann im Bereich der Ausnehmung 27 jeweils einen vorzugsweise gerundeten Vorsprung 28 aufweisen, der in die Ausnehmung 27 hineinfedert, wobei die Querschnittsgröße des Stecklochs 17 der Querschnittsgröße des Werkzeugschafts 5a entsprechen kann.

Bei diesen Ausführungsbeispielen ist das Werkzeug 5 aufgrund der Elastizität der Hülse 24a allseitig gegen die elastische Rückstellkraft F bewegbar oder nachgiebig, was wie bereits nach der Ausgestaltung nach Fig. 2 durch einen Kreuz-Doppelpfeil verdeutlicht ist.

Wie bereits erwähnt, kann der Arbeitskörper 5b bezüglich seiner Mittelachse quaderförmig bzw. prismatisch oder zylindrisch oder räumlich gerundet, z.B. kugelförmig, geformt sein. Grundsätzlich kann der Arbeitskörper 5b im Rahmen der Erfindung auch eine andere Form aufweisen. Es ist eine um seine Längsachse rotationssymmetrische Form des Arbeitskörpers 5b besonders vorteilhaft, weil der Behandler während der Behandlung unterschiedliche Drehstellungen um die Längsmittelachse des Arbeitskörpers 5b einnehmen kann, z.B. aus Gründen bequemerer Handhabung, ohne das Zwängungen im Bereich einer bereits teilweise eingeformten Kavität entstehen. Bei einem kugelförmigen Arbeitskörper 5b ist dieser vorgenannte Freiheitsgrad in vorteilhafter Weise in alle Bewegungsrichtungen, d.h. räumlich, gerichtet.

Die abrasive Arbeitsfläche 5c am Arbeitskörper 5b ist mit einer Vielzahl punktförmiger oder kantenförmiger Schneiden besetzt, die auf der jeweiligen Arbeitsfläche verteilt angeordnet sind. Hierbei kann es sich um geometrisch bestimmte oder unbestimmte Schneiden handeln. Es haben sich harte adhärente Körner, insbesondere aus Diamant, Feldspat oder Keramik als vorteilhaft erwiesen. Der Abstand der Schneiden voneinander kann gleich oder kleiner oder vorzugsweise größer als die Amplitude der Vibrationsbewegungen bemessen sein. Hierdurch erweist sich die Arbeitsfläche 5c im Vibrations- bzw. Schwingbetrieb als abrasiv wirksame Fläche, die einen zu bearbeitenden Gegenstand, z.B. einen Knochen, ein Modell oder einen Zahn, spanabhebend bearbeitet.

Der Arbeitskörper 5b ist bezüglich des Werkzeugschaftes 5a vorzugsweise kopfförmig verdickt. Ein um seine Mittelachse rotationssymmetrisch geformter Arbeitskörper 5b kann kugelförmig gemäß Fig. 3 bis 5 und 7 oder - in der Seitenansicht gesehen - in seiner Längsrichtung eiförmig oder gemäß Fig. 6 ellipsen- oder birnenförmig geformt sein.

Der Arbeitskörper 5b kann durch einen an sich als Rotations-Bohr- und Fräswerkzeug bekannten "Rosenbohrer" gebildet sein, der eine bezüglich seiner Mittelachse rotationssymmetrische Form aufweist und vorzugsweise kopfförmig verdickt ist und der den Fig. 5 bis 7 entsprechen kann. Ein Rosenbohrer weist durch rillenförmige Vertiefungen herausgearbeitete Schneidkanten 29 auf, die längs (Fig. 4 bis 6) und/oder quer (Fig. 3) und/oder jeweils S-förmig (Fig. 7) an der Oberfläche verlaufen können. Es hat sich bei Versuchen gezeigt, daß ein sogenannter Rosenbohrer sich auch bei einem Schwingantrieb vorzüglich eignet, wobei die Schneidkanten 29 schabend wirksam sind. Ein besonderer Vorteil ist darin zu sehen, daß die Schneidleistung bei einem Schwingantrieb an allen Stellen der Oberfläche des Arbeitskörpers 5b gleich ist, im Gegensatz zu einem Rotationsantrieb, bei dem die Schneidleistung in Richtung des Drehzentrums geringer wird und zwar aufgrund der sich in Richtung auf die Drehachse geringer werdenden Dreh- bzw. Winkelgeschwindigkeit. Bei der erfindungsgemäßen Ausgestaltung kann der Rosenbohrer darüberhinaus mit seinem Arbeitskörper 5b bezüglich seines Werkzeugschafts 5a seitlich versetzt und/oder abgewinkelt oder abgebogen sein, wodurch die Zugänglichkeit auch zu unzugänglichen Arbeitsstellen verbessert wird. Eine solche, seitlich versetzte oder seitlich ausgeformte Ausgestaltung des Arbeitskörpers 5b und/oder des Werkzeugsschaftes 5a ist bei einem Rotationswerkzeug oder Rotationsantrieb unbrauchbar, da ein solches Werkzeug im Rotationsbetrieb "seitlich schlagen" würde.

Bei allen vorbeschriebenen Ausführungsbeispielen ist es vorteilhaft, - wie bereits erwähnt - die Haltevorrichtung 4 so auszugestalten, daß der Werkzeugschaft 5a darin in mehreren stufenlos oder in Stufen verdrehten Stellungen fixierbar ist, so daß das Werkzeug 5 in wahlweisen verdrehten Stellungen einstellbar ist und individuell an vorhandenen Arbeitsstellen anpaßbar ist.

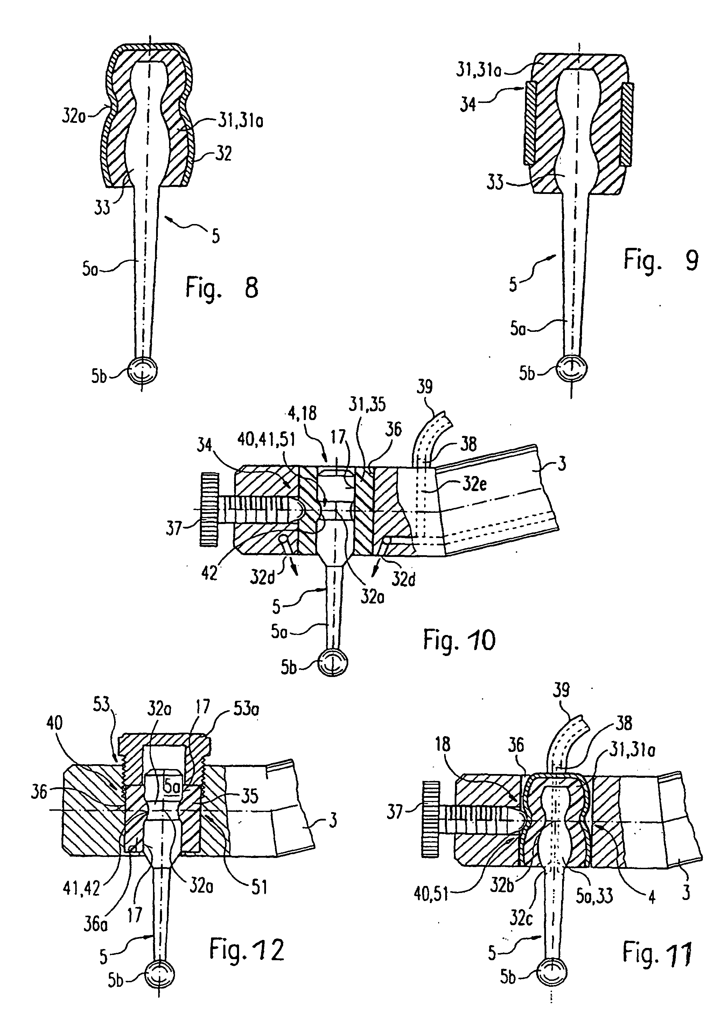

Bei der Ausgestaltung nach Fig. 8, bei der gleiche oder vergleichbare Teile mit gleichen Bezugszeichen versehen sind, ist ein hülsenförmiges Verbindungselement 31 aus elastisch verformbarem Material zwischen einer äußeren Schaftschale 32 und einem Schaftkern 33 angeordnet sein. Der Schaftkern 33 kann zylindrisch oder zwecks formschlüssigen Eingriffs mit dem Verbindungselement 31 unrund und/oder in seiner Längsrichtung eine von einer zylindrischen Form abweichende, also eine ungleich dicke Form, aufweisen, wodurch sich eine formschlüssige Verbindung mit dem Verbindungselement 31 ergibt. Dies ist auch zwischen dem Verbindungselement 31 und der äußeren Schaftschale 32 möglich. Bei der vorliegenden Ausgestaltung ist das Verbindungselement 31 ein den Hohlraum zwischen dem Schaftkern 33 und der Schaftschale 32 ausfüllendes und somit eingebettetes elastisches Füllmaterial, z. B. Gummi oder Kunststoff, das eine allseitig elastisch nachgiebige oder schwimmende Lagerung des Schaftkerns 33 in der Schaftschale 32 gewährleistet, und zwar radial, axial und in Umfangsrichtung. Beim Vorhandensein von erfindungsgemäßen Erhebungen und sie aufnehmenden Vertiefungen zwischen der Schaftschale 32 und dem Schaftkern 33, z.B. einer Ringnut in der Mantelfläche des Werkzeugschaftes 5a oder einer Taillierung der Schaftschale 32 und/oder des Schaftkerns 33 ist die formschlüssige Verbindung zwischen dem Verbindungselement 31 und der Schaftschale 32 einerseits und dem Schaftkern 33 andererseits gegeben, so daß es keiner weiteren Verbindung bedarf. Es ist jedoch möglich, das Verbindungselement 31 mit der Innenumfangsfläche der Schaftschale 32 und der Außenumfangsfläche des Schaftkerns 33 auf andere Weise fest zu verbinden, z.B. zu verkleben. Es ist möglich, das elastisch verformbare Material des Verbindungselements 31 zwischen den Schaftkern 33 und die Schaftschale 32 in flüssigem oder teigigem Zustand einzuspritzen, wodurch eine besonders einfache und schnelle Herstellung gewährleistet ist. Im Rahmen der Erfindung ist es weiter möglich, daß die Schaftschale 32 den Schaftkern 33 auch an der dem Arbeitskörper 5b zugewandten und/oder abgewandten Stirnseite überdeckt und an der jeweiligen Stirnseite zwischen der Schaftschale 32 und dem Schaftkern 33 das Verbindungselement 31 angeordnet ist. Es ist jedoch auch möglich, daß die Schaftschale 32, das Verbindungselement 31 und der Schaftkern 33 an dieser Stirnseite miteinander abschließen, und eine gemeinsame Stirnseite bilden. Bei dieser Ausgestaltung weist das Verbindungselement 31 die Form einer Hülse 31a oder Buchse auf. Die Schaftschale 32 kann eine Hülse oder Kappe aus Metall sein, wodurch der Werkzeugschaft 5a außen stabilisiert ist. Die von der vorbeschriebenen Halte- und Fixiervorrichtung 4, 18 ausgehenden Beanspruchungen können somit vom Werkzeugschaft 5a bei langer Lebensdauer aufgenommen werden. Beim Vorhandensein einer Ausnehmung 32a oder Ringnut kann ein darin einfassendes Sicherungselement wie eine Schraube eine formschlüssige und sonst kraftschlüssige Haltefunktion auf den Werkzeugschaft 5a ausüben. Die Querschnittsabmessung der Schaftschale 32 ist mit einem geringen Bewegungsspiel an die Querschnittsabmessung des Querlochs 17 angepaßt, so daß ein handhabungsfreundliches Einsetzen und Entfernen des Werkzeugs 5 möglich ist.

Im Rahmen der Erfindung ist es möglich und aus Vereinfachungsgründen auch vorteilhaft, den Werkzeugschaft 5a nur mit dem Schaftkern 33 und dem Verbindungselement 31 in Form einer elastischen Hülse auszuführen, d.h., die Schaftschale 32 wegzulassen. Auch eine solche Ausgestaltüng ist funktionsfähig. Ein solcher Werkzeugschaft 5b weist das hohlzylindrische Verbindungselement 31 als festes, z.B. angeklebtes, Anbauteil auf und kann mit einem geringen Bewegungsspiel und einer axialen Sicherung oder mit geringem Querschnitts-Übermaß hergestellt sein und somit in das Schaft-Aufnahmeloch 17 elastisch einpreßbar sein, wodurch aufgrund der elastisch radialen Spannkräfte ein fester Sitz und gegebenenfalls eine axiale Sicherung gewährleistet ist.

Es ist im Rahmen der Erfindung vorteilhaft, bei einer Feinbearbeitung mit einer größeren Dämpfungswirkung (große Elastizität) und bei einer Grobearbeitung mit einer geringeren Dämpfungswirkung (kleine Elastizität) zu arbeiten. Hierzu können Werkzeuge oder Handstücke unterschiedlich großer Dämpfung ausgebildet und bereitgestellt werden. Es ist auch vorteilhaft, das Dämpfungsmittel durch eine Einstellvorrichtung 34 so einstellbar auszugestalten, das es zwischen einer größeren und kleineren Dämpfung einstellbar ist.

Bei der Ausgestaltung nach Fig. 9, bei der gleiche oder vergleichbare Teile mit gleichen Bezugszeichen versehen sind, ist die Schaftschale 32 bezüglich ihrer Querschnittsgröße im Sinne einer Manschette oder Bandage veränderlich, wodurch die Elastizität des Dämpfungselements 31 veränderlich und einstellbar ist. Der Verstellmechanismus zum Verändern und Einstellen des in Fig. 9 mit 32a bezeichneten Schaftbandes ist nicht dargestellt. Es kann sich dabei um einen Verstellmechanismus handeln, wie er bei Schlauchklemmen üblich ist.

Bei der Ausgestaltung gemäß Fig. 10 ist das Verbindungselement 31 nicht ein Teil des Werkzeugs 5 sondern des Handstücks 2, hier des Handstückschaftes 3 und somit Teil der Antriebsverbindung für das Werkzeug 5. Bei der vorliegenden Ausgestaltung ist das Verbindungselement 31 eine Aufnahmehülse 35 aus elastischem Material wie Gummi oder Kunststoff, die in ein entsprechend bemessenes Querloch 36 im Handstückschaft 3 eingesetzt und darin befestigt ist, z. B. durch Kleben oder durch ein gegen die Aufnahmehülse 35 drückendes oder in sie teilweise einfassendes Sicherungselement 37, z.B. eine Schraube. Das Steckloch 17 ist bei dieser Ausgestaltung in der Aufnahmehülse 35 angeordnet, wobei es so groß bemessen sein kann, daß der Werkzeugschaft 5a mit einem geringen Bewegungsspiel oder mit einem geringen Übermaß und mit einer elastischen Preßspannung darin einsteckbar ist. Im ersten Fall und auch im zweiten Fall kann die Schraube die Funktion der axialen Sicherung des Werkzeugschaftes 5a dadurch übernehmen, daß sie radial nach innen gegen die Aufnahmehülse 35 drückt und diese verformt und dadurch eine kraftschlüssige oder bei einer Verformung in die Ausnehmung 32a oder Ringnut eine formschlüssige Sicherung des Werkzeugschaftes 5a gegen ein axiales Verschieben bewirkt.

In den Fig. 10 und 11 sind weitere Ausführungsbeispiele für eine Zuführung eines Kühl-, Spül- oder Behandlungsmediums zum Werkzeug 5a beschrieben, die ebenfalls bei allen vorbeschriebenen Ausgestaltungen realisierbar sind. Bei der Ausgestaltung nach Fig. 11 befindet sich das Werkzeug 5a nach Fig. 8 oder 9 im Aufnahmeloch 17, wobei das Sicherungselement 37 in die Ausnehmung 32a oder Ringnut einfaßt und eine axiale Sicherungsvorrichtung für das Werkzeug 5 bildet. Außerdem erstreckt sich ein axialer Kanalabschnitt 32b vom freien Schaftende bis in den Bereich wenigstens einer Arbeitsfläche 5c am Arbeitskörper 5b und zum dem Arbeitskörper abgewandten Stirnende des Werkzeugschaftes 5a, wobei an diesem Austrittsende ein lösbarer Leitungsanschluß 38 für eine außenseitig neben dem Handstückschaft 3 verlaufende Zuführungsleitung 39 für ein Kühl-, Spül- oder Behandlungsmedium vorgesehen ist. Das dem Arbeitskörper 5b zugewandte Ende kann mit einem oder mehreren radialen oder schrägen Kanalzweigen 32c im Übergangsbereich zum Arbeitskörper 5b oder in wenigstens einer Arbeitsfläche 5c ausmünden.

Bei der Ausgestaltung gemäß Fig. 10 ist eine oder sind mehrere Mündungsöffnungen 32d der Zuführungsleitung um das Steckloch 17 oder 36 verteilt angeordnet, die durch einen Umgehungskanal mit einem Schafthohlraum oder Zuführkanal in Verbindung stehen, der sich axial längs durch den Handstückschaft 3 erstrecken kann oder durch einen radialen Kanalzweig 32e und einem lösbaren Leitungsanschluß 38 mit der seitlich neben dem Handstückschaft 3 verlaufenden Zuführungsleitung 39 verbunden sein kann.

Es ist im Rahmen der Erfindung auch möglich, die Aufnahmehülse 35 in Verbindung mit der Ausnehmung 32a oder Ringnut im Werkzeugschaft 5a als axiale Sicherungsvorrichtung 40 und Verrastungsvorrichtung 41 auzubilden, wobei der Werkzeugschaft 5a mit geringem Bewegungsspiel in das Steckloch 17 einsteckbar ist und der Ausnehmung 32a oder Ringnut gegenüberliegend eine elastisch nachgiebige Verrastungsnase 42 vorgesehen ist, die in die Ausnehmung 32a oder Ringnut einfaßt und beim Ein- und Herausschieben des Werkzeugschaftes 5a manuell überdrückbar ist und beim Einschieben selbsttätig in die Ausnehmung 32a oder Ringnut expandiert.

Es ist im Rahmen der Erfindung möglich, die Verrastungsnase 42 als Vorsprung oder als Ring an die Innenwandung der Aufnahmehülse 35 anzuformen oder durch einen mit der Schraube auf die Aunahmehülse 35 erzeugten, radial einwärts gerichteten Druck nach innen auszubeulen. Die Festigkeit der Verrastungsvorrichtung 41 ist durch ein mehr oder weniger weites Einschrauben der Schraube einstellbar.

Mittels der Schraube 37 läßt sich auch die Elastizität des Verbindungselements 31 und somit die Rückstellkraft F verändern und einstellen. Die größte Elastizität ist dann gegeben, wenn die Schraube 37 oder die Verrastungsnase 42 nur geringfügig gegen die Aufnahmehülse 35 oder den Werkzeugschaft 5a drückt. Je mehr die Schraube 37 eingeschraubt wird und je größer der Anpreßdruck wird, desto geringer wird die Elastizität des Verbindungselements 31 und umgekehrt. Deshalb bildet die Schraube 37 in Verbindung der Aufnahme des Werkzeugschaftes 5a in der Aufnahmehülse 35 auch eine Einstellvorrichtung 5 zur Einstellung der Elastizität des Verbindungselements 31.

Es läßt sich auch eine freie Drehbarkeit des Werkzeugs 5 um seine Längsmittelachse in der Werkzeughalterung verwirklichen. Ein solches Drehgelenk 46 ist dann vorhanden, wenn der Werkzeugschaft 5a mit geringem radialem Bewegungsspiel im Aufnahmeloch 17 des Handstückschaftes 3 oder in der Aufnahmehülse 35 aufgenommen ist, so daß er darin frei drehbar ist und eine axiale Fixiervorrichtung vorhanden ist, die den Werkzeugschaft 5a zwar axial fixiert, jedoch nicht radial einwärts gegen ihn drückt. Dies vermag die als Vorsprung oder als Ring ausgebildete Verrastungsnase 42 durch ein Einfassen in die Ringnut 32a mit Bewegungsspiel zu erfüllen. Es ist möglich, die Verrastungsnase 42 innen anzuformen oder mittels der Schraube 37 soweit aus dem elastischen Material der Aufnahmehülse 35 herauszudrücken; daß sie die vorgenannten Merkmale erfüllt.

Es ist im weiteren möglich, daß die Schraube 37 die Aufnahmehülse 35 in einem Loch durchfaßt und mit ihrem freien, vorzugsweise gerundeten Ende in die Ringnut 32a einfaßt.

Eine freie oder etwas gebremste Drehbarkeit des Werkzeugs 5 hat den Vorteil, daß mit dem Handstück und mit dem Werkzeug 5 beim Drehen um seine Längsmittelachse kein schädliches Drehmoment auf den Zahn oder auf die Behandlungsstelle ausgeübt werden kann, da das Werkzeug 5 durchdreht. Hierdurch wird einerseits der Zahn oder das Zahnfleisch oder der Kieferknochen oder die Behandlungsstelle und andererseits das Werkzeug 5 selbst vor Überlastungen und Beschädigungen geschützt. Dies ist nicht nur bei solchen Werkzeugen 5 von Vorteil, deren Arbeitskörper 5a eine unrunde Querschnittsform aufweisen, mit der das Drehmoment besonders markant auf die Behandlungsstelle ausgeübt werden kann, sondern auch eine runde Querschnittsform aufweisen, insbesondere wenn es sich um einen dünnen Bearbeitungsabschnitt handelt, der zum Brechen oder Verbiegen neigt. Solche Werkzeuge 5 sind insbesondere dann gefährdet, wenn sie in der auszuarbeitenden Kavität festsitzen.

Die erfindungsgemäße Drehbarkeit des Werkzeugs 5 um seine Längsmittelachse in Verbindung mit dem Sicherungselemt 37 oder der Schraube bildet somit auch eine Überlast-Sicherungsvorrichtung 51, die aufgrund der einstellbaren Spannung eines vorzugsweise elastischen, radial einwärts gegen das Werkzeug 5 oder gegen den Werkzeugschaft 5a wirksamen Einstellelements, hier die Verrastungsnase 42, an das jeweils vorliegende, insbesondere durch die Festigkeit des Werkzeugs 5 vorgegebene Erforderdernis anpaßbar ist.

Desweiteren bildet das Sicherungselement 37 oder die Verrastungsnase 42 oder die Schraube eine Drehpositon-Feststellvorrichtung 53 für das Werkzeug 5, die es ermöglicht das Werkzeug 5 in bestimmten Drehpositionen einzustellen, und die hier durch das Ein- und Ausschrauben der Schraube 37, in und außer Funktion bringbar ist.

Beim Ausführungsbeispiel nach Fig. 12, bei dem gleiche oder vergleichbare Teile mit gleichen Bezugszeichen versehen sind, sind die axiale Sicherungsvorrichtung 40 und die Verrastungsvorrichtung 41 und ein Verbindungselement 31 mit einer abgewandelten Einstellvorrichtung 53 für die Elastizität. dem Handstück 2 bzw. dem Handstückschaft 3 zugeordnet. Bei dieser Ausgestaltung ist die elastische Aufnahmehülse 35 zwischen einem Widerlager, hier in Form einer Stufenfläche 36a des Aufnahmelochs 36 und einem Einstellelement 53a axial komprimierbar und somit mehr oder weniger expandierbar, so daß die Innenwandung des Aufnahmelochs 17 gegen den Werkzeugschaft 5a mehr oder weniger elastisch drückt und dadurch zum einen die Elastizität des Verbindungsmittels 31 und zum anderen die Größe des Drehmomentes bestimmt, bei dem die ebenfalls integrierte Überlast-Sicherungsvorrichtung 51 in Funktion tritt und der Werkzeugschaft 5a durchdrehen kann. Das Einstellelement 53a kann durch eine in das Aufnahmeloch 36 eingeschraubte Handmutter mit Griffrillen gebildet sein. Die Verrastungsvorrichtung 41 ist durch eine innere elastische Verrastungsnase 42 in Form eines Vorsprungs oder Rings gebildet, der in vorbeschriebener Weise beim Ein- und Ausschieben des Werkzeugschaftes 5b überdrückbar ist.

## Patentansprüche

1. Medizinisches Instrument (1),
a) mit einem Handstück (2), an dessen vorderem Ende ein Werkzeug (5) mit einem Werkzeugschaft (5a) und einem daran angeordneten Arbeitskörper (5b) durch eine Haltevorrichtung (4) lösbar mit dem Handstück (2) verbunden ist,
b) wobei das Handstück (2) einen Handstückschaft (3) aufweist, der durch einen Schwingungserreger (6) in Schwingungen versetzbar ist,
c) wobei der Werkzeugschaft (5a) durch ein hülsenförmig ausgebildetes und den Werkzeugschaft (5a) umgebendes Verbindungselement (24)
d) aus elastisch verformbarem Material mit dem Handstückschaft (3) verbunden ist,
**dadurch gekennzeichnet,**
e) dass am Werkzeugschaft (5a) wenigstens ein radialer Vorsprung (26) oder wenigstens eine radiale Ausnehmung (27) ausgebildet ist,
f) wobei der Vorsprung (26) sich elastisch in die Wandung des Verbindungselements (24) hineindrückt oder der Werkstoff des Verbindungselements (24a) sich elastisch in die Ausnehmung (27) hineindrückt.

2. Instrument nach Anspruch 1,
**dadurch gekennzeichnet,**
**dass** der Vorsprung (26) und die Ausnehmung (27) gerundet sind.

3. Instrument nach Anspruch 1 oder 2,
**dadurch gekennzeichnet,**
**dass** der Vorsprung (26) und die Ausnehmung (27) sich ringförmig auf dem gesamten Umfang des Werkzeugschaftes (5a) erstrecken.

4. Instrument nach einem der vorherigen Ansprüche,
**dadurch gekennzeichnet,**
**dass** zwei Vorsprünge (26) oder Ausnehmungen (27) axial hintereinander angeordnet sind.

5. Instrument nach einem der vorherigen Ansprüche,
**dadurch gekennzeichnet,**
**dass** das hülsenförmige Verbindungselement (24) an seinem hinteren Ende einen Hülsenboden (24c) aufweist.

6. Instrument nach einem der vorherigen Ansprüche,
**dadurch gekennzeichnet,**
**daß** der Werkzeugschaft (5a) in einer Anordnung längs oder quer zum Handstückschaft (3) mit diesem verbunden ist.

7. Instrument nach einem der vorherigen Ansprüche,
**dadurch gekennzeichnet,**
**dass** der Handstückschaft (3) nach vorne absteht.

8. Instrument nach einem der vorherigen Ansprüche,
**dadurch gekennzeichnet,**
**daß** der Handstückschaft (3) sich gerade erstreckt oder in seinem vorderen Endbereich abgewinkelt ist, z. B. um einen Winkel (W) von etwa 5 bis 60°, insbesondere etwa 15°.

## Claims

1. Medical instrument (1),
(a) having a handpiece (2) at the forward end of which a tool (5), having a tool shaft (5a) and a working body (5b) arranged thereon, is releasably connected with the handpiece (2) by means of a mounting device (4),
(b) the handpiece (2) having a handpiece shaft (3) which can be set into oscillation by means of an oscillation generator (6),
(c) the tool shaft (5a) being, by means of a connection element (24) formed to be sleeve-shaped and surrounding the tool shaft (5a)
(d) and of elastically deformable material, connected with the handpiece shaft (3),
**characterised in that**,
(e) there is formed on the tool shaft (5a) at least one radial projection (26) or at least one radial recess (27),
(f) the projection (26) pressing itself elastically into the wall of the connection element (24) or the material of the connection element (24a) pressing itself elastically into the recess (27).

2. Instrument according to claim 1,
**characterised in that**,
the projection (26) and the recess (27) are rounded.

3. Instrument according to claim 1 or 2,
**characterised in that**,
the projection (26) and the recess (27) are annular and extend over the entire circumference of the tool shaft (5a).

4. Instrument according to any preceding claim,
**characterised in that**,
two projections (26) or recesses (27) are arranged axially behind one another.

5. Instrument according to any preceding claim,
**characterised in that**,
the sleeve-shaped connection element (24) has a sleeve floor (24c) at its rearward end.

6. Instrument according to any preceding claim,
**characterised in that**,
the tool shaft (5a) is, in an arrangement longitudinal or transverse of the handpiece shaft (3), connected therewith.

7. Instrument according to any preceding claim,
**characterised in that**,
the handpiece shaft (3) stands out forwardly.

8. Instrument according to any preceding claim,
**characterised in that**,
the handpiece shaft (3) extends straight or is angled in its forward end region, e.g. by an angle (W) from about 5 to 60°, in particular about 15°.

## Revendications

1. Instrument médical (1)
a) ayant une pièce à main (2) à l'extrémité antérieure de laquelle un outil (5), comportant une tige d'outil (5a) et un corps de travail (5b) disposé sur celle-ci, est relié, de façon détachable, à la pièce à main (2) par un dispositif de retenue (4),
b) la pièce à main (2) présentant une tige de pièce à main (3) qui peut être mise en vibrations par un générateur de vibrations (6),
c) la tige d'outil (5a) étant reliée, par un élément de liaison (24) en forme de manchon entourant la tige d'outil (5a),
d) et constitué d'une matière déformable élastiquement à la tige de la pièce à main (3),
**caractérisé en ce que**,
e) sur la tige d'outil (5a), est formée au moins une saillie radiale (26) ou au moins un creux radial (27),
f) la saillie (26) se pressant de manière élastique à l'intérieur de la paroi de l'élément de liaison (24) ou bien la matière de l'élément de liaison (24a) se pressant de manière élastique à l'intérieur du creux (27).

2. Instrument selon la revendication 1,
**caractérisé en ce que**,
la saillie (26) et le creux (27) sont arrondis.

3. Instrument selon la revendication 1 ou 2,
**caractérisé en ce que**
la saillie (26) et le creux (27) s'étendent de façon annulaire sur toute la périphérie de la tige d'outil (5a).

4. Instrument selon l'une quelconque des revendications précédentes,
**caractérisé en ce que**,
deux saillies (26) ou creux (27) sont disposé(e)s axialement l'un(e) derrière l'autre.

5. Instrument selon l'une quelconque des revendications précédentes,
**caractérisé en ce que**,
l'élément de liaison (24) en forme de manchon présente un fond de manchon (24c) à son extrémité postérieure.

6. Instrument selon l'une quelconque des revendications précédentes,
**caractérisé en ce que**,
la tige d'outil (5a), dans une disposition longitudinale ou transversale à la tige de la pièce à main (3), est reliée à celle-ci.

7. Instrument selon l'une quelconque des revendications précédentes,
**caractérisé en ce que**,
la tige de la pièce à main (3) dépasse vers l'avant.

8. Instrument selon l'une quelconque des revendications précédentes,
**caractérisé en ce que,**
la tige de la pièce à main (3) est rectiligne ou recourbée dans sa zone terminale avant, par exemple, avec un angle (W) d'environ 5 à 60°, en particulier environ 15°.
